# EUROPÄISCHE PATENTANMELDUNG

(11) **EP 2 562 240 A1**
(43) Veröffentlichungstag der Anmeldung: **27.02.2013**
(21) Anmeldenummer: 12005719.5
(22) Anmeldetag: 07.08.2012
(51) Int. Cl.: C12M 1/00

(54) **Laboratoriums-Brutschrank mit verbesserter Innenraum-Befeuchtung**

(30) Priorität: 24.08.2011 DE 102011111754
(71) Anmelder: Thermo Electron LED GmbH, 63505 Langenselbold (DE)
(72) Erfinder: Hohenthanner, Ulrike Dr., 63457 Hanau (DE); Pieczarek, Waldemar, 63505 Langenselbold (DE); Stahl, Hermann, 61130 Nidderau-Ostheim (DE)
(74) Vertreter: Tomerius, Isabel

(57) **Zusammenfassung**

Die Erfindung betrifft einen Laboratoriums-Brutschrank (1), insbesondere Begasungs-Brutschrank, mit einem von einem Gehäuse (2) umgebenen Innenraum (3) und einem außerhalb des Innenraums (3) angeordneten Dampferzeuger (4), der mit dem Innenraum (3) über eine Dampfzuleitung (5) in Verbindung steht. Der Dampferzeuger (4) ist als im Wesentlichen druckloser Behälter (40) mit einem Bodenbereich (41) und einem darüberliegenden Dampfraum (42) ausgebildet, wobei der Bodenbereich (41) zur Aufnahme eines Wasserreservoirs (6) ausgebildet und mit einer Heizvorrichtung (7) zur Erwärmung des Wasserreservoirs (6) versehen ist, die Dampfzuleitung (5) den Dampferzeuger (4) im Bereich des Dampfraumes (42) verlässt und eine Luftzuleitung (8) in den Dampferzeuger (4) einmündet, um dem Dampferzeuger (4) Umgebungsluft zuzuführen und mit Wasserdampf angereicherte Luft über die Dampfzuleitung (5) im Wesentlichen drucklos in den Innenraum (3) des Laboratoriums-Brutschranks (1) einzuleiten. Die Erfindung betrifft außerdem ein Verfahren zum Betreiben eines solchen Laboratoriums-Brutschranks.

## Beschreibung

Die Erfindung betrifft einen Laboratoriums-Brutschrank, auch als Inkubator bezeichnet, insbesondere einen Begasungs-Brutschrank, mit einem von einem Gehäuse umgebenden Innenraum und einem außerhalb des Innenraums angeordneten Dampferzeuger, der mit dem Innenraum über eine Dampfzuleitung in Verbindung steht.

Laboratoriums-Brutschränke dienen in Laboratorien üblicherweise dazu, in ihrem Innenraum Proben, insbesondere biologische und/oder mikrobiologische Proben, unter vorgegebenen Bedingungen wie einer bestimmten Temperatur und Luftfeuchtigkeit und - im Falle von Begasungsbrutschränken - einer definierten Gasatmosphäre zu lagern. Meist wird dabei versucht, die Bedingungen des menschlichen oder tierischen Körpers zu imitieren. Oft gewählte Bedingungen sind daher eine Temperatur von ca. 37 °C und eine möglichst hohe Luftfeuchtigkeit, die in der Regel bei mindestens 60 %, bevorzugt bei mindestens 80 %, besonders bevorzugt bei mindestens 90 % liegen sollte, ohne dass dabei jedoch an den Wänden oder anderen Bereichen des Laboratoriums-Brutschrankes Feuchtigkeit auskondensiert.

Im Stand der Technik sind verschiedene Möglichkeiten bekannt, in einem Laboratoriums-Brutschrank eine feuchte Innenraumatmosphäre zu erzeugen. Eine erste Möglichkeit besteht darin, im Inneren des Laboratoriums-Brutschrankes ein Wasserreservoir vorzusehen, aus dem durch Beheizen Wasser verdampft wird (zum Beispiel EP 1552888 A2). Ein großes Problem dieser Lösung ist jedoch die leichte Verkeimung des Wasserbades und die Gefahr der Kontamination der im Laboratoriums-Brutschrank gelagerten Proben. Diese Verkeimungsgefahr kann deutlich reduziert werden, wenn dem Innenraum des Laboratoriums-Brutschranks von außerhalb überhitzter Wasserdampf zugeführt wird. Bekannt sind zum Beispiel Lösungen, in denen außerhalb des Laboratoriums-Brutschranks ein Autoklav oder Sterilisator aufgestellt ist, aus dem überhitzter Wasserdampf unter Druck in den Laboratoriums-Brutschrank zugeführt wird. Diese Lösung ist jedoch aufwendig und teuer. Zudem unterliegen diese Geräte den Sicherheitsanforderungen für Druckbehälter, in Deutschland beispielsweise der Betriebssicherheitsverordnung. Ein weiterer Nachteil besteht darin, dass aufgrund der Heißdampf-Zufuhr in den Innenraum des Laboratoriums-Brutschranks ein sehr hoher, stoßartiger Wärmeeintrag erfolgt, der es schwierig macht, eine konstante Temperatur innerhalb des Laboratoriums-Brutschrankes einzuhalten. Außerdem lässt sich auch kaum vermeiden, dass bei der Zufuhr des Dampfes unter Druck Wasser in den Innenraum des Laboratoriums-Brutschranks spritzt und sich unter Umständen auf den dort gelagerten Proben niederschlägt.

Es bestand also ein Bedarf an einem Laboratoriums-Brutschrank, in dessen Innenraum mit geringem Aufwand eine gleichmäßig hohe Feuchtigkeit und eine konstante Temperatur eingehalten werden kann und die Gefahr der Kontamination möglichst gering gehalten wird. Aufgabe der Erfindung ist es, einen solchen Laboratoriums-Brutschrank anzugeben.

Die Lösung dieser Aufgabe gelingt mit dem Laboratoriums-Brutschrank gemäß Anspruch 1. Bevorzugte Weiterbildungen des Laboratoriums-Brutschranks sind in den zugehörigen Unteransprüchen beschrieben. Die Erfindung betrifft weiterhin ein Verfahren zum Betreiben eines derartigen Laboratoriums-Brutschrankes.

In einem ersten Aspekt betrifft die Erfindung also einen Laboratoriums-Brutschrank, vorzugsweise einen Begasungs-Brutschrank, mit einem Gehäuse, das einen Innenraum umgibt, und einem außerhalb des Innenraums angeordneten Dampferzeuger, der über eine Dampfzuleitung mit dem Innenraum in Verbindung steht. Der Dampferzeuger ist als im Wesentlichen druckloser Behälter mit einem Bodenbereich und einem darüber liegenden Dampfraum ausgebildet. Der Bodenbereich kann ein Wasserreservoir aufnehmen, das mit einer Heizvorrichtung erwärmt werden kann. In den Dampferzeuger mündet eine Luftzuleitung ein, um dem Dampferzeuger Umgebungsluft zuzuführen. Im Inneren des Dampferzeugers wird diese Umgebungsluft mit Wasserdampf angereichert und über eine Dampfzuleitung, die den Dampferzeuger im Bereich des Dampfraumes verlässt, im Wesentlichen drucklos in den Innenraum des Laboratoriums-Brutschranks eingeleitet.

Im Unterschied zum Stand der Technik, in dem Wasserdampf im Inneren des Laboratoriums-Brutschrankes erzeugt wird, ist in der vorliegenden Erfindung ein externer Dampferzeuger vorhanden. Damit wird die Anwesenheit eines verkeimenden Wasserbades im Inneren des Laboratoriums-Brutschrankes vermieden. Von den externen Dampferzeugern des Standes der Technik, die dem Innenraum des Laboratoriums-Brutschrankes überhitzten Wasserdampf unter Druck zuführen, unterscheidet sich die Erfindung dadurch, dass es sich bei dem Dampferzeuger um einen im Wesentlichen drucklosen Behälter handelt und der Wasserdampf im Wesentlichen drucklos in den Innenraum des Laboratoriums-Brutschrankes eingeleitet wird. Bei dem erfindungsgemäß eingesetzten Dampferzeuger handelt es sich also nicht um einen Druckbehälter, in dessen Innerem gezielt ein Überdruck zur Bildung überhitzten Wasserdampfes erzeugt wird. Der erfindungsgemäß eingesetzte Dampferzeuger unterfällt nicht den einschlägigen Vorschriften für Druckbehälter oder Dampfkessel wie beispielsweise der Betriebssicherheitsverordnung oder deren Vorgängern Druckbehälter- und Dampfkesselverordnung. Konkret wird also während des Betriebs des Dampferzeugers kein oder nur ein sehr geringer Überdruck erzeugt, der in jedem Fall bei maximal 0,5 bar, vorzugsweise unter 0,2 bar, liegt. Zudem wird während des Betriebs des Dampferzeugers auch kein überhitzter Wasserdampf erzeugt, sondern die Temperatur der mit Wasserdampf angereicherten Luft und des Wasserreservoirs liegen in jedem Fall unterhalb der Siedetemperatur des Wassers, also in jedem Fall unter 100 °C und vorzugsweise bei maximal 90 °C. Der im erfindungsgemäß verwendeten Dampferzeuger gebildete Wasserdampf ist kein komprimierter Dampf, sondern wird im Wesentlichen drucklos (maximal 0,5 bar, vorzugsweise unter 0,2 bar) in den Innenraum des Laboratoriums-Brutschrankes eingeleitet, da der Wasserdampf in einem im Wesentlichen drucklosen Behälter erzeugt wird und keine Maßnahmen zur Komprimierung des Wasserdampfes getroffen werden. Zweckmäßig ist vielmehr an wenigstens einer Stelle des vom Wasserdampf durchströmten Bereiches, bevorzugt im Laboratoriums-Brutschrank, eine Druckausgleichsöffnung vorhanden.

Die Zufuhr im Wesentlichen unkomprimierten Wasserdampfes hat den Vorteil, dass der Wärmeeintrag in den Innenraum des Laboratoriums-Brutschrankes wesentlich gleichmäßiger erfolgen kann, als dies bei Zuleitung von komprimiertem Heißdampf der Fall ist. Da innerhalb des Dampferzeugers kein Hochdruck erzeugt werden muss, sind auch keine abschließenden Ventile erforderlich, und diese sind bevorzugt in der Dampfzuleitung auch nicht vorhanden. Ein stoßweises Zuführen von überhitztem Wasserdampf nach Öffnen der Ventile in den Innenraum des Laboratoriums-Brutschrankes wird daher vermieden. Vielmehr kann der Wasserdampf gleichmäßiger in den Innenraum zugeführt werden. Außerdem ist die Temperatur des zugeführten Wasserdampfes geringer als im Fall von überhitztem Heißdampf, so dass der Temperatureintrag pro Zeiteinheit geringer ist und gleichmäßiger erfolgen kann. Dies erleichtert die Einhaltung einer Temperaturkonstanz im Innenraum des Laboratoriums-Brutschrankes erheblich. Die geringeren Schwankungen der Temperatur und Feuchtigkeit erlauben es zudem, den Feuchtegehalt im Innenraum insgesamt höher zu setzen als im Stand der Technik, da die Gefahr der Überfeuchtung und damit Auskondensation von Wasser deutlich geringer ist. Damit verringert sich in vorteilhafter Weise auch die Gefahr eines Austrocknens der Proben.

Die Verwendung von Ventilen zum Abschließen von Hochdruck-Dampferzeugern im Stand der Technik führt außerdem regelmäßig dazu, dass nach dem Öffnen der Ventile und der Zufuhr von Heißdampf in den Innenraum des Laboratoriums-Brutschrankes Wassertropfen in den Innenraum spritzen. Auch dieses Problem tritt bei der vorliegenden Erfindung nicht auf, da keine Ventile zum Abschließen des Dampferzeugers gegenüber dem Laboratoriums-Brutschrank benötigt werden. Ein weiterer Vorteil der im Wesentlichen drucklosen Erzeugung von Wasserdampf besteht darin, dass Zuleitungen mit einem erheblich größeren Querschnitt als im Falle der Dampfdruckbehälter eingesetzt werden können. Gerade im Bereich der Engstellen und Ventile innerhalb der Zuleitungen besteht im Stand der Technik die Gefahr, dass es hier zu Verunreinigungen durch Keimbildung kommt. Dieses Problem wird in der vorliegenden Erfindung ebenfalls vermieden, da Zuleitungen mit größeren Querschnitten ohne Verwendung von Ventilen wesentlich weniger zur Verkeimung und dem Aufwachsen von so genanntem Biofouling und damit einhergehenden Verstopfungen neigen. Außerdem wurde überraschend festgestellt, dass es zur Verhinderung der Keimbildung im Wasserreservoir des Dampferzeugers nicht erforderlich ist, das Wasser auf mindestens 100 °C zu erhitzen. Vielmehr ist es zur Vermeidung der Keimbildung bereits ausreichend, wenn das Wasserbad zumindest 60 °C besitzt. Noch besser lässt sich eine Keimbildung verhindern, wenn das Wasserreservoir auf mindestens 70 °C, vorzugsweise auf mindestens 80 °C, erwärmt und besonders bevorzugt bei 85 bis 90 °C gehalten wird. Wie bereits erwähnt, sollte es erfindungsgemäß nur vermieden werden, dass das Wasserbad zu sieden beginnt.

Bei ausreichender Erwärmung des Wasserreservoirs im Dampferzeuger bildet sich oberhalb des Wasserreservoirs im Dampfraum eine stark mit Wasserdampf angereicherte Atmosphäre. Bevorzugt ist es, wenn der Dampfraum mit Wasserdampf gesättigt ist. In diesem Fall kann die in den Dampferzeuger eingeleitete Umgebungsluft sich rasch mit Wasser anreichern, und diese in den Innenraum des Laboratoriums-Brutschranks eingeleitete, mit Wasserdampf angereicherte Luft führt dort sehr schnell zu einer starken Befeuchtung der Innenraumatmosphäre, da die im Laboratoriums-Brutschrank eingestellte Innentemperatur von beispielsweise 37 °C deutlich niedriger liegt als die Temperatur der dampfgesättigten Luft, die dem Innenraum aus dem Dampferzeuger zugeführt wird. Die zugeführte, mit Wasserdampf gesättigte Luft gibt also ihre Feuchtigkeit sehr rasch an die Innenraumatmosphäre des Laboratoriums-Brutschrankes ab.

Um die in den Dampferzeuger eingeleitete Umgebungsluft mit Wasserdampf anzureichern, ist es üblicherweise ausreichend, wenn die Luftzuleitung im Bereich des Dampfraumes in den Dampferzeuger einmündet. Normalerweise reicht es aus, wenn dafür gesorgt wird, dass die zugeleitete Umgebungsluft eine hinreichend lange Strecke durch den Dampfraum des Dampferzeugers zurücklegen muss, bevor sie den Dampferzeuger durch die Dampfzuleitung wieder verlässt. Zweckmäßig liegen Luftzuleitung und Dampfzuleitung also möglichst weit voneinander entfernt. Eine noch bessere Anreicherung der eingeleiteten Umgebungsluft mit Wasserdampf kann erreicht werden, wenn die Luftzuleitung im Bodenbereich in den Dampferzeuger mündet, so dass die Umgebungsluft durch das Wasserreservoir hindurch in den darüber liegenden Dampfraum und von dort durch die Dampfzuleitung in den Laboratoriums-Brutschrank gelangt. Diese Dampfzuleitung verlässt den Dampferzeuger zweckmäßig in einem oberen Bereich des Dampfraumes. Um zu verhindern, dass eventuell in der Dampfzuleitung kondensiertes Wasser in den Innenraum des Laboratoriums-Brutschrankes läuft, wird die Dampfzuleitung vorzugsweise vom Dampferzeuger in Richtung auf den Laboratoriums-Brutschrank hin ansteigend angeordnet. In diesem Fall läuft auskondensiertes Wasser aus der Dampfzuleitung in den Dampferzeuger zurück.

Zur Förderung der Luft innerhalb der erfindungsgemäßen Vorrichtung kann im Prinzip jedes geeignete Fördermittel eingesetzt werden. Ausreichend sind sehr einfache und kostengünstige Fördermittel wie beispielsweise ein Gebläse oder eine Pumpe. Bevorzugt ist es dabei, das Fördermittel in einem Bereich anzuordnen, wo die Luft noch kalt und nicht mit erhitztem Wasserdampf angereichert ist, um das Fördermittel nicht der erhöhten Temperatur und Feuchtigkeit auszusetzen. Zweckmäßig ist es daher, die Luftzuleitung in einem Bereich, bevor diese in den Dampferzeuger eintritt, mit dem Fördermittel, also beispielsweise der Pumpe oder dem Gebläse, zu verbinden. Um zu verhindern, dass Verunreinigungen mit der Umgebungsluft in den Dampferzeuger und von dort in den Laboratoriums-Brutschrank gelangen, wird dem Fördermittel bevorzugt ein Filter, insbesondere ein Sterilfilter, vor- oder bevorzugt nachgeschaltet.

In einem Begasungs-Brutschrank, zum Beispiel einem CO₂-Inkubator, wird zusätzlich zu Temperatur und Feuchtigkeit auch noch eine bestimmte Gasatmosphäre im Innenraum des Laboratoriums-Brutschrankes eingestellt. Die hierfür verwendeten Gase werden nachfolgend zur Unterscheidung von der ebenfalls in den Laboratoriums-Brutschrank eingeleiteten, mit Wasserdampf angereicherten Luft als Prozessgase bezeichnet. Die Verwendung derartiger Prozessgase in Begasungsbrutschränken ist grundsätzlich bereits bekannt. Beispielsweise wird Kohlendioxid zum Einstellen eines bestimmten pH-Wertes innerhalb des Begasungs-Brutschrankes verwendet, Stickstoff zur Erzeugung einer Inertgasatmosphäre und Sauerstoff, um die Bedingungen sauerstoffreichen Bluts zu imitieren und Oxidationsvorgänge zu fördern. Damit diese Prozessgase in den Innenraum des Laboratoriums-Brutschrankes gelangen können, ist zusätzlich wenigstens eine Gaszuleitung vorgesehen. Diese Gaszuleitung kann beispielsweise unmittelbar in den Innenraum des Laboratoriums-Brutschrankes einmünden. Da die Prozessgase üblicherweise in getrockneter Form zur Verfügung stehen, reduziert sich bei deren Einleiten die Feuchtigkeit im Innenraum des Laboratoriums-Brutschrankes. Dies wiederum kann zur Folge haben, dass die Steuerung des Laboratoriums-Brutschrankes für eine erhöhte Zufuhr mit Wasserdampf beladener Umgebungsluft sorgt, um den vorgegebenen Feuchtigkeitsstand im Innenraum wiederherzustellen. Dies führt dann zur Verdrängung des eingeleiteten Prozessgases, das gegebenenfalls durch weitere Einleitung wieder auf die gewünschte Konzentration angehoben werden muss. So kommt ein unerwünschter Regelkreislauf mit unnötig erhöhten Gaszufuhren in Gang. Erfindungsgemäß kann dies dadurch umgangen werden, dass anstelle des trockenen Prozessgases angefeuchtetes Prozessgas in den Innenraum des Laboratoriums-Brutschrankes eingeleitet wird. Zu diesem Zweck ist es bevorzugt, dass die Gaszuleitung in den Dampferzeuger einmündet, um das Prozessgas im Dampfraum anzufeuchten, bevor es dann in den Innenraum des Laboratoriums-Brutschrankes weitergeleitet wird. In einer besonders bevorzugten Weiterbildung der Erfindung ist eine Gaszuleitung für Prozessgas vorhanden, die sowohl in den Dampferzeuger als auch in den Innenraum des Laboratoriums-Brutschrankes einmündet. Besonders zweckmäßig sind zusätzlich Mittel vorhanden, beispielsweise wenigstens ein Ventil, um das Prozessgas wahlweise dem Dampferzeuger oder dem Innenraum zuzuführen. Auf diese Weise ist es möglich, abhängig von dem für den Innenraum gemessenen Feuchtigkeitsgehalt das Prozessgas entweder, wenn der Feuchtigkeitsgehalt im Innenraum sehr hoch ist, als trockenes Prozessgas unmittelbar in den Innenraum einzuleiten oder, wenn der Feuchtigkeitsgehalt unterhalb eines vorgegebenen Schwellenwertes liegt, das Prozessgas dem Dampferzeuger zuzuführen, damit es dort mit Feuchtigkeit angereichert wird und es dann in den Innenraum des Begasungs-Brutschrankes gelangt. Die Weiterleitung des Prozessgases aus dem Dampferzeuger in den Begasungs-Brutschrank erfolgt zweckmäßig über die Dampfzuleitung, über die auch angefeuchtete Umgebungsluft in den Begasungs-Brutschrank geleitet wird.

Die Messung der Betriebsparameter innerhalb des Laboratoriums-Brutschrankes erfolgt grundsätzlich auf im Stand der Technik bekannte Art und Weise. Die Erfindung hat den Vorteil, dass der grundsätzliche Aufbau des Laboratoriums-Brutschranks und dessen Betrieb sowie die Steuerung der Verfahrensabläufe wie im Stand der Technik erfolgen können. Kostenintensive Umbaumaßnahmen sind nicht erforderlich. Von Vorteil kann es sein, wenn vor oder während des Betriebs des Laboratoriums-Brutschrankes der Umgebungsdruck bei der Einstellung der Temperatur des Wasserreservoirs im Dampferzeuger berücksichtigt wird. Dies ist sinnvoll, da der Umgebungsdruck, der vom Aufstellungsort des Laboratoriums-Brutschrankes abhängt, einen Einfluss auf die Siedetemperatur des Wassers hat, ein Sieden des Wasserreservoirs erfindungsgemäß jedoch vermieden werden soll. In einer besonders einfachen Variante wird dabei die Höhe des Aufstellungsortes mit der maximalen Temperatur des Wasserreservoirs korreliert. Beispielsweise kann vorgesehen sein, dass man die Höhe des Aufstellungsortes vor Inbetriebnahme des erfindungsgemäßen Laboratoriums-Brutschrankes in die Steuerung des Gerätes einprogrammiert, die dann wiederum dafür sorgt, dass die für die Aufstellungshöhe entsprechende Maximaltemperatur des Wasserreservoirs nicht überschritten wird, oder die Maximaltemperatur des Wasserreservoirs wird unmittelbar vorgegeben. Alternativ kann der Umgebungsdruck mit einem in das Gerät eingebauten Druckmesser, beispielsweise einem in einen Infrarot-Kohlendioxid-Sensor integrierten Drucksensor, gemessen und die maximale Wassertemperatur automatisch ermittelt und eingehalten werden.

Durch Einstellen einer ausreichend hohen Temperatur im Wasserreservoir kann einerseits eine wasserdampfgesättigte Atmosphäre im Dampfraum des Dampferzeugers sichergestellt und andererseits dafür gesorgt werden, dass im Wasserreservoir keine Kontamination auftritt. Die großen Leitungsquerschnitte und wenigen Engstellen innerhalb des erfindungsgemäßen Gerätes verhindern eine Kontamination zusätzlich. Dennoch sind gelegentliche Desinfektionsverfahren im Verlauf der Lebensdauer des erfindungsgemäßen Laboratoriums-Brutschrankes in der Regel erforderlich, und diese sind mit dem Gerät besonders leicht durchzuführen. Besonders bevorzugt wird für die Desinfektion trockene heiße Luft verwendet, die eine Temperatur von beispielsweise mindestens 140 °C und besonders bevorzugt etwa 180 °C aufweist. Diese heiße Luft kann im Dampferzeuger erzeugt und von dort in den Laboratoriums-Brutschrank eingeleitet werden. Hierzu wird zweckmäßig zunächst das Wasser des Wasserreservoirs verdampft, wozu die Temperatur über die normale Betriebstemperatur hinaus erhöht und das Wasserbad zum Sieden gebracht wird. Die auf mindestens 140 °C erwärmte trockene Luft entsteht nach dem vollständigen Verdampfen des Wasserreservoirs. Es sind also keinerlei zusätzliche Gerätschaften zur Durchführung des Desinfektionsschrittes erforderlich. Alternativ kann die Heißluft aus einem externen Heißlufterzeuger zugeführt werden.

Im Vorstehenden wurde die Erfindung so beschrieben, dass für einen Laboratoriums-Brutschrank ein Dampferzeuger vorgesehen ist. Es ist jedoch ebenfalls möglich, mehrere Brutschränke mit einem Dampferzeuger zu verbinden. Zu diesem Zweck führt aus dem Dampferzeuger entweder mehr als eine Dampfzuleitung heraus, nämlich pro Laboratoriums-Brutschrank jeweils eine Dampfleitung, oder aber eine aus dem Dampferzeuger herausführende Dampfzuleitung wird auf mehrere Brutschränke aufgeteilt. Zweckmäßig ist dann in jeder der Dampfzuleitungen ein Ventil vorhanden, das gesondert angesteuert werden kann, so dass die Dampfzufuhr zu jedem der Brutschränke individuell eingestellt werden kann. Die Verwendung nur eines Dampferzeugers für mehrere Brutschränke senkt den Platzbedarf und die Kosten für die Gesamtanordnung. Handelt es sich bei den Brutschränken um Begasungsbrutschränke, sind die Gaszuleitungen analog den Dampfzuleitungen so ausgebildet, dass die Gaszufuhr zu jedem der Brutschränke individuell eingestellt werden kann.

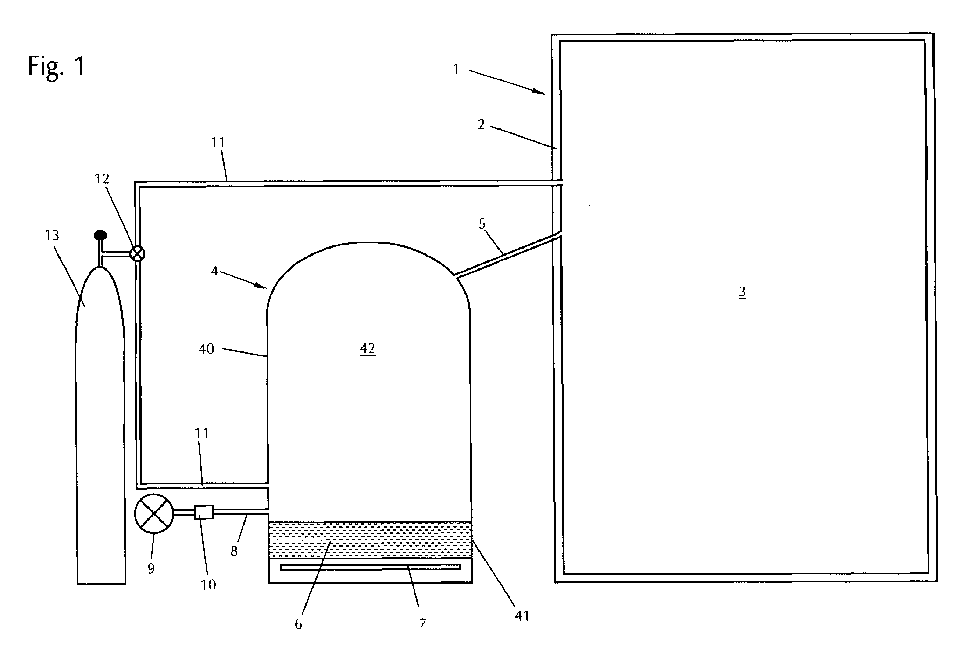

Die Erfindung soll nachfolgend anhand einer Zeichnung näher erläutert werden. In der schematischen und nicht maßstabsgerechten Zeichnung zeigt
- Fig. 1: einen Querschnitt durch einen erfindungsgemäßen Laboratoriums-Brutschrank mit angeschlossenem Dampferzeuger.

Figur 1 beschreibt die Erfindung am Beispiel eines Begasungs-Brutschrankes (Inkubators) 1. Der Inkubator 1 umfasst ein Gehäuse 2, das einen Innenraum 3 umschließt, in dem zum Beispiel mikrobiologische Proben unter vorgegebenen Temperatur-, Feuchtigkeits- und Gasatmosphären-Bedingungen gelagert werden können. Übliche Lagerbedingungen für die Proben sind beispielsweise eine Temperatur von 37 °C und eine Luftfeuchtigkeit von etwa 95 %. Der Innenraum 3 ist durch eine im Querschnitt nicht erkennbare Tür verschließbar. Die Innenausstattung wie beispielsweise Tragböden, Messeinrichtungen usw. ist zur Vereinfachung weggelassen.

Zur Erzeugung der im Innenraum 3 benötigten Feuchtigkeit ist mit dem Inkubator 1 ein Dampferzeuger 4 über eine Dampfzuleitung 5 verbunden. Der Dampferzeuger 4 besteht aus einem Behälter 40 mit einem Bodenbereich 41, der ein Wasserreservoir 6 aufnehmen kann, und einem darüber liegenden, hier kuppelförmig ausgebildeten Dampfraum 42. Bei dem Behälter 40 handelt es sich um einen drucklosen Behälter - im Unterschied zu Dampfkesseln oder Druckbehältern, in denen bei Betrieb ein Überdruck von mehr als 0,5 bar herrscht. Im erfindungsgemäßen Dampferzeuger 4 dagegen wird Wasserdampf, der durch Erhitzen des Wasserreservoirs 6 mittels einer Heizvorrichtung 7 erzeugt wird, im Dampferzeuger 4 nicht komprimiert, so dass der Dampf im Wesentlichen dem Umgebungsdruck entspricht und in jedem Fall bei höchstens 0,5 bar, üblicherweise unter 0,2 bar, liegt.

Der im Dampfraum 42 vorhandene Wasserdampf wird dem Inkubator 1 dadurch zugeführt, dass mittels einer Pumpe 9 Luft aus der Umgebung des Dampferzeugers 4 über die Luftzuleitung 8 in das Innere des Dampferzeugers 4 gefördert wird. Im gezeigten Fall mündet die Luftzuleitung 8 oberhalb des Wasserreservoirs 6 in den Dampfraum 42. Es ist alternativ auch möglich, die Luftzuleitung tiefer zu legen, sodass die Umgebungsluft durch das Wasserreservoir hindurch in den Dampfraum 42 geführt wird. Im Dampferzeuger reichert sich die Umgebungsluft mit dem Wasserdampf an und gelangt über die Dampfzuleitung 5 in den Innenraum 3 des Inkubators. Damit mit der Umgebungsluft keine Verunreinigungen in den Dampferzeuger 4 gefördert werden, ist zwischen Pumpe 9 und Dampferzeuger 4 in der Luftzuleitung 8 ein Sterilfilter 10 angeordnet. Die Anordnung der Pumpe 9 vor dem Dampferzeuger 4 hat den Vorteil, dass keine heiße und feuchte Luft durch die Pumpe geführt werden muss. Die Pumpe 9 kann deshalb sehr einfach ausgebildet sein.

Das Wasserreservoir 6 wird mittels der Heizvorrichtung 7 vorzugsweise auf eine Temperatur von mindestens 60 °C, besonders bevorzugt auf 85 bis 90 °C erhitzt. Je höher die Temperatur des Wasserreservoirs 6, desto geringer die Wahrscheinlichkeit, dass sich Keime im Wasser bilden. Die Temperatur des Wasserbades sollte jedoch nicht so hoch sein, dass das Wasser im Reservoir 6 zu sieden beginnt. Oberhalb des Wasserreservoirs 6 bildet sich eine mit Wasserdampf gesättigte, ebenfalls etwa 90 °C heiße Atmosphäre. In dieser reichert sich die zugeführte Umgebungsluft sehr rasch und in hohem Maße mit Wasserdampf an und erwärmt sich auf eine Temperatur, die deutlich oberhalb der im Innenraum 3 des Inkubators herrschenden Temperatur von 37 °C liegt. Daher gibt die durch die Dampfzuleitung 5 zugeführte feuchtwarme Luft sehr rasch Feuchtigkeit an die Atmosphäre im Innenraum 3 des Inkubators 1 ab. Im Unterschied zu der herkömmlichen Zuführung von überhitztem Wasserdampf unter Druck erfolgt die Zufuhr hier jedoch nicht stoßweise, sondern kontinuierlich. Außerdem ist die Temperatur der feuchten Luft geringer als im Fall von überhitztem Wasserdampf. Diese vorteilhaften Eigenschaften erlauben eine sehr viel einfachere und konstantere Temperaturregulierung im Innenraum des Inkubators. Außerdem wird das Einsprühen von Wassertropfen in den Innenraum 3 vermieden, da im erfindungsgemäßen Gerät keine Ventile in der Dampfzuleitung 5 benötigt werden, so dass es auch nicht zu einem Verspritzen von Wasser bei der Dampfzufuhr kommt. Ein weiterer Vorteil gegenüber der Dampferzeugung in Druckgefäßen besteht darin, dass die Zuleitungsquerschnitte erheblich größer sein können als im Fall des Standes der Technik. Dies verhindert die Kontamination der Zuleitungen, da keine Engstellen vorhanden sind, in denen sich Keime festsetzen könnten. Sollte dennoch einmal Wasser in der Dampfzuleitung 5 kondensieren, verhindert die schräg in Richtung auf den Innenraum 3 ansteigende Anordnung der Zuleitung, dass dieses Kondensationswasser in den Innenraum 3 gelangt. Vielmehr läuft es in Richtung auf den Dampferzeuger 4 zurück.

Zur Erzeugung einer inerten Atmosphäre ist außerhalb des Innenraums 3 eine Stickstoff-Druckflasche 13 angeordnet, die über Gaszuleitungen 11 sowohl mit dem Dampferzeuger 4 als auch mit dem Innenraum 3 des Inkubators 1 verbunden ist. Über ein Ventil 12 kann ausgewählt werden, ob der Stickstoff dem Dampferzeuger 4 oder dem Innenraum 3 zugeführt werden soll. Bevorzugt geschieht diese Auswahl in Abhängigkeit von den Bedingungen, die für den Innenraum 3 gemessen werden. Die Erfassung der Messewerte, deren Auswertung und die Steuerung des Gerätes erfolgen wie bereits im Stand der Technik üblich. In an sich bekannter Weise erfolgt eine Gaszufuhr immer dann, wenn die gemessene Gaskonzentration im Innenraum 3 einen vorgegebenen Schwellenwert unterschreitet. Weitere Werte, die während des Betriebs des Inkubators gemessen werden, sind üblicherweise die Temperatur und der Feuchtegehalt der Innenraumatmosphäre. Bevorzugt bestimmt der für die Feuchtigkeit im Innenraum 3 ermittelte Istwert, ob Stickstoff aus der Gasflasche 13 unmittelbar in den Innenraum 3 eingeleitet wird oder über den Dampferzeuger 4. Da es sich bei dem zugeführten Prozessgas in der Regel um ein getrocknetes Gas handelt, führt dessen unmittelbare Zuleitung in den Innenraum 3 zu einer Absenkung der Feuchtigkeit. Ist die im Innenraum 3 gemessene Feuchtigkeit daher geringer als ein vorgegebener Schwellenwert, ist es bevorzugt, mit Feuchtigkeit angereichertes Gas in den Innenraum 3 einzuleiten. In diesem Fall also wird aus der Gasflasche 13 durch entsprechende Schaltung des Ventils 12 das Gas durch den Dampfraum 42 des Dampferzeugers 4 hindurch in den Innenraum 3 geleitet. Auf dem Weg dorthin reichert es sich mit dem im Dampferzeuger 4 gebildeten Wasserdampf an und wird so als feuchtes Gas in den Innenraum 3 zugeführt. Eine unerwünschte weitere Absenkung der Feuchtigkeit im Innenraum 3 kann auf diese Weise verhindert werden. Gleiches wie hier für die Zuleitung von Stickstoff geschildert, gilt auch für andere Prozessgase wie Kohlendioxid und Sauerstoff. Auch in diesem Fall kann die Zudosierung in Abhängigkeit von der im Innenraum gemessenen Feuchtigkeit wahlweise direkt oder über den Dampferzeuger erfolgen.

## Patentansprüche

1. Laboratoriums-Brutschrank (1), insbesondere Begasungs-Brutschrank, mit einem von einem Gehäuse (2) umgebenen Innenraum (3) und einem außerhalb des Innenraums (3) angeordneten Dampferzeuger (4), der mit dem Innenraum (3) über eine Dampfzuleitung (5) in Verbindung steht,
**dadurch gekennzeichnet,**
**dass** der Dampferzeuger (4) als im Wesentlichen druckloser Behälter (40) mit einem Bodenbereich (41) und einem darüberliegenden Dampfraum (42) ausgebildet ist,
wobei der Bodenbereich (41) zur Aufnahme eines Wasserreservoirs (6) ausgebildet und mit einer Heizvorrichtung (7) zur Erwärmung des Wasserreservoirs (6) versehen ist,
die Dampfzuleitung (5) den Dampferzeuger (4) im Bereich des Dampfraumes (42) verlässt und eine Luftzuleitung (8) in den Dampferzeuger (4) einmündet, um dem Dampferzeuger (4) Umgebungsluft zuzuführen und mit Wasserdampf angereicherte Luft über die Dampfzuleitung (5) im Wesentlichen drucklos in den Innenraum (3) des Laboratoriums-Brutschranks (1) einzuleiten.

2. Laboratoriums-Brutschrank nach Anspruch 1,
**dadurch gekennzeichnet,**
**dass** die Luftzuleitung (8) im Bereich des Dampfraumes (42) in den Dampferzeuger (4) einmündet.

3. Laboratoriums-Brutschrank nach Anspruch 1,
**dadurch gekennzeichnet,**
**dass** die Luftzuleitung (8) im Bodenbereich (41) in den Dampferzeuger (4) so einmündet, dass in den Dampferzeuger (4) gelangte Umgebungsluft durch das Wasserreservoir (6) hindurchgeleitet wird.

4. Laboratoriums-Brutschrank nach einem der Ansprüche 1 bis 3,
**dadurch gekennzeichnet,**
**dass** die Dampfzuleitung (5) vom Dampferzeuger (4) zur Einmündung in den Innenraum (3) hin ansteigt.

5. Laboratoriums-Brutschrank nach einem der Ansprüche 1 bis 4,
**dadurch gekennzeichnet,**
**dass** die Luftzuleitung (8) außerhalb des Dampferzeugers (4) mit einer Pumpe (9) oder einem Gebläse verbunden ist.

6. Laboratoriums-Brutschrank nach Anspruch 5,
**dadurch gekennzeichnet,**
**dass** der Pumpe (9) oder dem Gebläse ein Sterilfilter (10) vor- oder nachgeschaltet ist.

7. Laboratoriums-Brutschrank nach einem der Ansprüche 1 bis 6,
**dadurch gekennzeichnet,**
**dass** wenigstens eine Gaszuleitung (11) zum Einleiten eines Prozessgases, insbesondere Kohlendioxid, Stickstoff oder Sauerstoff, in den Innenraum (3) vorgesehen ist.

8. Laboratoriums-Brutschrank nach Anspruch 7,
**dadurch gekennzeichnet,**
**dass** die Gaszuleitung (11) auf eine der folgenden Weisen ausgebildet ist:
- die Gaszuleitung (11) mündet in den Innenraum (3),
- die Gaszuleitung (11) mündet in den Dampferzeuger (4), um Prozessgas von dort in den Innenraum (3) weiterzuleiten, oder
- die Gaszuleitung (11) mündet sowohl in den Dampferzeuger (4) als auch den Innenraum (3), und es sind bevorzugt Mittel, insbesondere wenigstens ein Ventil (12), vorhanden, um das Prozessgas wahlweise dem Dampferzeuger (4) oder dem Innenraum (3) zuzuführen.

9. Verfahren zum Betreiben eines Laboratoriums-Brutschranks (1), insbesondere eines Begasungs-Brutschranks, nach einem der Ansprüche 1 bis 8,
**dadurch gekennzeichnet,**
**dass** es die Schritte umfasst:
- Betreiben der Heizvorrichtung (7), um das Wasserreservoir (6) zu erwärmen und im Dampfraum (42) eine mit Wasserdampf angereicherte Atmosphäre zu erzeugen,
- Einleiten von Umgebungsluft in den Dampferzeuger,
- Durchleiten der Luft durch den Dampfraum (42), um die Luft mit Wasserdampf anzureichern, und
- Zufuhr der mit Wasserdampf angereicherten Luft durch die Dampfzuleitung (5) in den Innenraum (3).

10. Verfahren nach Anspruch 9,
**dadurch gekennzeichnet,**
**dass** das Wasserreservoir (6) auf eine Temperatur unterhalb des Siedepunktes des Wassers, aber auf mindestens 60 °C, bevorzugt mindestens 70 °C, besonders bevorzugt mindestens 80 °C, insbesondere auf eine Temperatur von 85 bis 90 °C aufgeheizt wird.

11. Verfahren nach Anspruch 9 oder 10,
**dadurch gekennzeichnet,**
**dass** die Temperatur des Wasserreservoirs (6) in Abhängigkeit vom Umgebungsdruck, vorzugsweise unter Berücksichtigung der Höhe des Aufstellungsortes des Laboratoriums-Brutschrankes (1), eingestellt wird.

12. Verfahren nach einem der Ansprüche 9 bis 11,
**dadurch gekennzeichnet,**
**dass** die Einleitung von Prozessgas durch den Dampferzeuger (4) in den Innenraum (3) erfolgt, wenn im Innenraum (3) eine Feuchtigkeitswert ermittelt wird, der unterhalb eines vorgegebenen Schwellenwertes liegt, und das Prozessgas direkt in den Innenraum (3) eingeleitet wird, wenn der im Innenraum (3) ermittelte Feuchtigkeitswert wenigstens gleich dem Schwellenwert ist.

13. Verfahren nach einem der Ansprüche 9 bis 12,
**dadurch gekennzeichnet,**
**dass** außerhalb des regulären Betriebs ein Desinfektionsschritt durchgeführt wird, indem zunächst das Wasser des Wasserreservoirs (6) verdampft wird und anschließend trockene, erhitzte Umgebungsluft durch den Dampferzeuger (4), die Dampfzuleitung (5) und den Innenraum (3) geleitet wird, wobei die trockene Umgebungsluft vorzugsweise auf mindestens 140 °C, besonders bevorzugt auf etwa 180 °C, erhitzt wird.
